# EUROPEAN PATENT APPLICATION

(11) **EP 1 932 436 A1**
(43) Veröffentlichungstag der Anmeldung: **18.06.2008**
(21) Anmeldenummer: 05784135.5
(22) Anmeldetag: 31.08.2005
(51) Int. Cl.: A23L 1/29, A61K 36/06, C12N 1/14, C12R 1/77

(54) **BIOLOGISCH AKTIVES NAHRUNGSMITTELPRODUKT AUF MILCHBASIS**

(71) Anmelder: Obschestvo S Ogranichennoi Otvetstvennostyu Gella-, Moscow 105215 (RU)
(72) Erfinder: GRIGORASH, Alexandr Iliich, Moscow, 105568 (RU); MAKLANOV, Anatoly Ivanovich, Moscow, 105568 (RU); SAMOILENKO, Vladimir Alexandrovich, Moskovskaya obl., 142290 (RU); OKUNEV, Oleg Nikolaevich, Moskovskaya obl., 142290 (RU)
(74) Vertreter: Jeck, Anton
(86) Internationale Anmeldenummer: PCT/RU2005/000442
(87) Internationale Veröffentlichungsnummer: WO 2007/027121

(57) **Zusammenfassung**

Die Erfindung betrifft ein biologisches Produkt, insbesondere Nahrungsmittel, welches bei der Züchtung des Pilzes der Art Fusarium erzeugt wird. Das Produkt enthält Kulturflüssigkeit und Biomasse, die bei der Züchtung der Pilze der Art Fusarium sambucinum auf Sauer- oder Labmolke oder fettarmer Milch erzeugt wird. E3ei standardisierter Feuchtigkeit von 6,5 % und einem Fettgehalt von 10 - 15 % wird eine Fett- und Säurezusammensetzung erreicht, die biologisch aktive Stoffe enthält, die als Produkt zur Stärkung der Immunität des menschlichen Körpers und zur Heilung und Prophylaxe allergischer Erkrankungen verwenden werden kann.

## Beschreibung

Die Erfindung betrifft ein biologisch aktives Produkt, insbesondere Nahrungsmittel, welches bei der Züchtung des Pilzes der Art Fusarium erzeugt wird. Biologisch aktive Nahrungsmittel auf Milchbasis und auf Basis der Milchsäurebakterien sind weit bekannt [1, 2, 3]. Solche Produkte werden mit Hilfe der Milchsäuregärung und Milchlaktoseverwertung erzeugt.

Der Mangel der bekannten Nahrungsmittel besteht darin, dass die Endprodukte bis zu 3 % Äthanol enthalten. Der weitere Mangel ist, dass das Spektrum der biologisch aktiven Stoffe, die während der Züchtung der Bakterien produziert werden, begrenzt ist. Deshalb können die Milchsäureprodukte nur bei bestimmten Erkrankungen des Magen-Darm-Kanals, bei chronischer Pankreatitis und bei Lebererkrankungen verwendet werden.

Bekannt ist ein Milchsäuregetränk auf Molicebasis. Das Getränk wird mit Hilfe der Kefirpilze unter Belüftungsbedingungen gegärt [4]. Für die Zubereitung des Getränks werden Kefirpilze unter aeroben Bedingungen auf einem Nährboden auf Basis der eiweißarmen Molke kultiviert. Die Molke (whey) ist durch fettarme Milch und Äthanol angereichert.

Der Vorteil dieses Produktes im Vergleich zum traditionellen Kefir besteht im reduzierten Eiweißgehalt im Medium vor der Fermentierung, in der Durchsichtigkeit des Fertiggetränks, sowie in der Vereinfachung der Sterilisierung des Fertigprodukts. Aber das Getränk ist dem traditionellen Kefir nach dem Gehalt an biologisch aktiven Stoffen und nach seiner Einwirkung auf das Immunsystem wesentlich unterlegen.

In letzter Zeit werden für die Herstellung der biologisch aktiven Nahrungsmittel oft die Pilze Fusarium sambucinum verwendet. Diese Pilze können Laktose verwerten und ein breites Spektrum der biologisch aktiven Stoffe, Vitamine, Fermente, Kofermente, Glykane usw. produzieren. Das weist eine heilsame Wirkung auf den Magen-Darm-Kanal und die Leber auf und kräftigt das Immunsystem.

Bekannt ist ein Eiweißfutterzusatz (protein food additive), welcher durch eine tiefe Züchtung des Pilzes Fusarium sambucinum auf der verdünnten Molke mit späterer Abtrennung der Pilzbiomasse erzeugt wird. Der Zusatz wird als Futterzusatz für Tiernahrung verwendet, um sie mit Ubichinon (ubiquinone) anzureichern [6].

Der Mangel des bekannten Zusatzes besteht darin, dass wenig Pilzbiomasse angesammelt wird. Darüber hinaus gehen die biologisch aktiven Stoffe, die in der Kulturflüssigkeit (culture liquid) produziert werden, bei der Abtrennung der Pilzbiomasse verloren. Außerdem ist der bekannte Zusatz für Menschen nicht geeignet.

Bekannt ist eine Zusammensetzung, die den Gewebemetabolismus des Menschen beeinflusst. Für diese Zusammensetzung wird der Pilzstamm Fusarium sambucinum verwendet - Handelsname "Milaif" [7]. Die Zusammensetzung hat einen Satz der essentiellen Aminosäuren, ein Komplex der Glykane (glicanes), die immunomodulierende Wirksamkeit aufweisen, Phospholipide und ungesättigte Fettsäuren (free fatty), die die Detoxikationseigenschaften der Leber verstärken, sowie Vitamine D, E, Ubichinon Q10, die starke Antioxidanten sind.

Der Mangel dieses Präparats besteht darin, dass ein Teil der biologisch aktiven Stoffe bei der Entfernung der Kulturflüssigkeit verloren geht.

Bekannt sind biologisch aktive Nahrungszusätze (Ausführungsformen), die biologisch aktive Stoffe enthalten. Diese werden bei der Züchtung des Pilzgeflechts Fusarium sambucium aus Biomasse oder Kulturflüssigkeit gewonnen [8]. Die immunomodulierende Eigenschaften dieses Produkts, ihre prophylaktische Wirkung auf den Zustand des Magens und der Leber sind durch klinische Versuche bewiesen [5].

Der Mangel dieser Produkte besteht in relativ hohem Aufwand im Zusammenhang mit der Zubereitung des Nährbodens für die Züchtung. Des Weiteren ist auch die Notwendigkeit der zusätzlichen Kosten für die Verarbeitung der Biomasse und der Kulturflüssigkeit und für die Entfernung der biologisch aktiven Stoffe ein Nachteil.

Das biologisch aktive Produkt, welches durch die aerobe Züchtung des Pilzstammes Fusarium culmorum WSB auf Milchbasis gewonnen wird, liegt dem vorgeschlagenen biologisch aktiven Nahrungsprodukt besonders nahe. Das Produkt wird zur Heilung und Prophylaxe der allergischen Erkrankungen verwendet [9].

Der Mangel des bekannten Produkts besteht darin, dass der Gehalt an wertvollen biologisch aktiven Stoffen, beispielsweise Fetten, niedrig ist. Der weitere Mangel besteht darin, dass die Erzeugung der biologisch aktiven Produkte durch Erzeugerstämme eingeschränkt ist, und dass das Produkt für die Prophylaxe der Erkrankungen des Magen-Darm-Kanals nicht verwendet werden kann.

Es ist Aufgabe der Erfindung ein biologisch aktives Produkt, insbesondere Nahrungsmittel, zu entwickeln, das einen niedrigen Gehalt an Laktose und einen erhöhten Gehalt an wertvollen biologisch aktiven Verbindungen hat, und daher für die Kräftigung des Immunsystems des Menschen und die Wiederherstellung der Funktion des Magen-Darm-Kanals und der Leber verwendet werden kann.

Diese Aufgabe der Erfindung wird dadurch gelöst, dass es Kulturflüssigkeit und Biomasse enthält, die bei der Züchtung der Pilze der Art Fusarium sambucinum auf Sauer- oder Labmolke oder fettarmer Milch erzeugt wurden und bei der Feuchtigkeit von 6,5 % einen Fettgehaft von 10 - 15 % aufweist, wobei die Fett- und Säurezusammensetzung in % von der Summe der Fette beträgt:
α-Linolensäure 0,2 - 0,4 %, γ- Linolensäure 0,4 - 0,7 %, Eikosantriensäure 0,2 - 0,35 %, Arachidonsäure 0,4 - 0,8 %;
Proteine 24 - 35 %, darunter in % der Summe der Proteine Aminosäure 16 - 24%, Laktose 1 - 3 % und Asche 6 - 8 % sowie Vitamine 300 - 500 mg/100 g, darunter in mg/100 g Vitamin E 6 - 8 mg/100 g, Vitamin B1 0,6 - 0,9 mg/100 g, Vitamin B2 4 - 6 mg/100 g, Vitamin B3 3,5 - 6,5 mg/100 g und Vitamin B4 250 - 400 mg/100 g.

Die Eiweiß-Kohlenhydrate der fettarmen Milch der Molke (Lab- oder Sauermolke) werden umgewandelt in ein neues biologisch aktives Produkt, welches einen erhöhten Gehalt an biologisch aktiven Stoffen und ein verbreitetes physiologisches Wirkungsspektrum hat. Das Produkt kann für einen breiten Personenkreis für die Prophylaxe des metabolischen Syndroms bei Störungen des Magen-Darm-Kanals, sowie für die Kräftigung des Immunsystems des Menschen verwendet werden.

Das biologisch aktive Produkt kann dadurch gewonnen werden, dass es als Nahrungsmittel Kulturflüssigkeit und Biomasse enthält, das bei der Züchtung der Stämme des Pilzes der Art Fusarium sambucinum aus einem der Reihe Fusarium sambucinum ASM F-26109D, F-166D-WSB-916(PS-64), F-169D-WSB-917, F-30510, F-3052D, F-8427D, F-199D-52587 oder F-676D-139S genommen wird.

Als Weiterbildung kann das biologisch aktive Produkt auch dadurch gewonnen werden, dass es von der Biomasse abgetrennte Kulturflüssigkeit enthält, die bei der Züchtung der Pilze der Art Fusarium sambucinum auf Sauer- oder Labmolke oder auf fettarmer Milch erzeugt wurde und dabei folgende Stoffe aufweist: Enniatin 100 - 800 mg/l und Fermente: Kollagenasen 100 - 800 U/ml, Proteasen 1 - 2 U/I sowie Pektinasen 0,4 - 0,8 U/ml.

Das neue Produkt ist dann dadurch gekennzeichnet, dass es Kulturflüssigkeit enthält, die bei der Züchtung der Stämme des Pilzes der Art Fusarium sambucinum aus einem der Reihe Fusarium sambucinum ASM F-26109D, F-165D-WSB-916(PS-64), F 169D-WSB- 917, F-3051D, F-3052D, F-8427D, F- 199D-52587 oder F-676D-139S genommen wird.

Dabei sind die Stämme des Pilzes der Art Fusarium sambucinum, die bei der Zubereitung des biologisch aktiven Nahrungsmittels als Erzeuger der biologisch aktiven Stoffe verwendet wurden, bekannt und werden in der Allrussischen Sammlung der Iridustrlemikroorganismen des Forschungsinstitutes "WNII Genetika" und in der Allrussischen Sammlung der Mikroorganismen von IBAN AN UdSSR hinterlegt.

Die verwendete fettarme Milch ist ein Nebenerzeugnis bei der Herstellung von Sahne und Butter und kann nur eingeschränkt als Rohstoff in der Süßwarenindustrie verwendet werden.

Die Molke ist ein Abfallprodukt der Milchindustrie bei der Herstellung des Quarks und der Käse. Molke kann als Nahrungsmittel nicht verwendet werden, weil sie wenig Proteine und Fette und viel Laktose enthält. Deshalb wird Molke für die Reinigung abgelassen, oder teilweise als Laktosequelle für die Herstellung von Arzneimitteln verwendet. Allgemein bekannt ist das Problem einer effektiven Entsorgung der Molke.

Um die fettarme Milch und Molke als wertvolles Nahrungsmittel verwenden zu können, müssen diese verarbeitet werden, wobei die Konzentration der Laktose reduziert wird und sie mit biologisch aktiven Stoffen angereichert werden.

Die Erfindung wird anhand einiger Beispiele veranschaulicht. Diese Beispiele sind durch die Ansprüche dieser Erfindung erfasst.

### Beispiel 1 (Ausführungsform 1)

Für die Zubereitung des biologisch aktiven Nahrungsmittels wird die Sauermolke nach der Vakuumtrocknung verwendet. Die Molke hat folgende physikalische und chemische Werte: Gewichtsanteil in %, Fett - 0,4, Eiweiß - 5,8, Feuchtigkeit - 6,5, Laktose - 70,8, Asche - 11. Die Verdünnung der Molke bei der Züchtung wird nach der Laktose auf 4,5% standardisiert.

Pilzgeflecht Fusarium sambucinum ASM F-3051D wird im 10-Liter-Fermenter ANKUM 2M in 7 L der Molke mit der Zugabe des Saatguts (seed material) 5,6 g/l, des salpetersaueren Ammoniums (ammonium sulfat NH4NO3) (0,3 %), schwefelsauren Magniums (magnesium sulfate MaSO4 x 7H2O) (0,5 %) und saueren phosphorsauren Kaliums (potassium phosphate dibasic KH2PO4) (0,2 %) bei Temperatur 26 °C, Umrührung (300 ppm), Aeration (2 1 Luft/min) innerhalb von 54 Stunden gezüchtet.

Das Saatgut, dessen Erzeuger der Stamm Fusarium sambucinum ASM F-3051D ist, wird folgenderweise erzeugt:
- Suspension der 10 Tage alten Kultur Fusarium sambucinum auf Kartoffel-Möhre-Agar (Chlamidosporen) wird im 250 ml-Kolben mit 30 ml 3,5 °B der Würze (malt) besät. Die Züchtung wird auf einer Schwengelmaschine mit 220 ppm unter 27 °C innerhalb von 3 Tagen durchgeführt. Es werden Makrokonieiden (Saatgut Nr. 1) gewonnen, die für die Züchtung des Saatguts Nr. 2 verwendet werden.
- Saatgut Nr. 1 in der Menge von 3 - 5 % wird in den 250 mi-pColben mit 30 ml 3,5 °B der Würze besät. Die Züchtung wird auf einer Schwengelmaschine mit 220 ppm unter 27 °C innerhalb von 3 Tagen durchgeführt (Saatgut Nr. 2). Das gewonnene Saatgut stellt ein dichtes Pilzgeflecht aus Flockenkolonien dar. Das Saatgut Nr. 2 wird als flüssige Subkultur (10 % des Volumens) für die Aussaat der Industriekultur im Fermenter verwendet.

Nach der Züchtung innerhalb von 45 Stunden wird das optimale Produkt - 12,9 g/l gewonnen. Die gewonnene Biomasse und Kulturflüssigkeit auf Molkebasis wird eingedickt, bis der Feuchtigkeitsanteil 6,5 % beträgt. Das fertige biologisch aktive Nahrungsmittel hat bei einer Feuchtigkeit von 6,5 % folgenden Gehalt in Gew, %: Fette - 10,4, wobei die Fett- und die Säurezusammensetzung beträgt (in % von der Summe der Fette) - Linolsäure - 56,92, α - Linolensäure - 0,31,y - Linolensäure - 0,58, Eikosantriensäure - 0,27, Arachidonsäure - 0,66; Proteine - 30,8, darunter Aminosäuren (in % von der Summe der Proteine) - 17,0; Laktose - 2,5; Asche - 7,1; sowie Vitamine - 473,21 mg/100 g, darunter Vitamin E - 7,2 mg/100 g, Vitamin B1 - 0,7 mg/100 g, Vitamin B2 - 4,9 mg/100 g, Vitamin B3 - 5,4 mg/100 g und Vitamin B4 -303,5 mg/100 g.

Das biologisch aktive Nahrungsmittel kann als selbstständiges Produkt, sowie als Zusatz bei der Zubereitung jeglicher Nahrungsmittel verwendet werden.

### Beispiel 2

Dasselbe wie im Beispiel 1, aber bei der Zubereitung des biologisch aktiven Nahrungsmittels wird als Milchbasis I,abmolke und als Pilz der Art Fusarium sambucinum der Stamm Fusarium sambucinum ASM F-3052D verwendet. Das fertige Produkt stellt Kulturflüssigkeit und Biomasse dar, die bei einer Feuchtigkeit von 6,5 % folgenden Gehalt in Gew. % hat: Fette - 14,5, wobei Fett- und Säurezusammensetzung beträgt (in % von der Summe der Fette) - Linolsäure - 57,3, α Linolensäure - 0,34, γ - Linolensäure - 0,6, Eikosantriensäure - 0,32, Arachidonsäure - 0,7; Proteine - 28,5, darunter Aminosäuren (in % von der Summe der Proteine) - 16,4; Laktose - 2,45; Asche - 6,6; sowie Vitamine - 430,1 mg/100 g, darunter Vitamin E - 6,9 mg1100 g, Vitamin B1 - 0,68 mg/100 g, Vitamin B2 - 4,9 mg/100 g, Vitamin B3 - 5,0 mg/100 g und Vitamin B4 -285 mg/100 g.

### Beispiel 3

Dasselbe wie im Beispiel 1, aber bei der Zubereitung des biologisch aktiven Nahrungsmittels wird fettarme Milch, die nach dem Laktosegehalt im Nährboden vor der Saat (3 - 6 %) standardisiert ist, und als Pilz der Art Fusarium sambucinum der Stamm Fusarium sambucinum F-676D-139S verwendet. Das fertige Produkt stellt Kulturflüssigkeit und Biomasse dar, die bei einer Feuchtigkeit von 6,5 % folgenden Gehalt haben, Gew. in %: Fette - 12,5, wobei die Fett- und die Säurezusammensetzung beträgt (in % von der Summe der Fette) - Linolsäure - 55,35, α- Linolensäure - 0,36, γ-Linolensäure - 0,48, Eikosantriensäure - 0,3, Arachidonsäure - 0,54; Proteine - 31,2, darunter Aminosäuren (in % von der Summe der Proteine) - 17,8; Laktose - 3,0; Asche - 6,5; sowie Vitamine - 450,15 mg/100 g, darunter Vitamin E - 6,5 mg/100 g, Vitamin B1 - 0,6 mg/100 g, Vitamin B2 - 4,3 mg/100 g, Vitamin B3 - 4,8 mg/100 g und Vitamin B4 -310 mg/100 g.

### Beispiel 4 (Ausführungsform 2)

Für die Zubereitung des biologisch aktiven Nahrungsmittels wird die Sauermolke nach der Vakuumtrocknung verwendet. Die Molke hat folgende physikalische und chemische Werte: Gewichtsanteil in %: Fett - 0,4, Eiweiß - 5,8, Feuchtigkeit - 6,5, Laktose - 70,8, Asche - 11. Die Verdünnung der Molke bei der Züchtung wird auf Laktose - 4,5 % standardisiert.

Pilzgeflecht Fusarium sambucinum ASM F-3051D wird im 10-Liter-Fermenter ANKUM 2M in 7 L der Molke mit der Zugabe des Saatguts 5,6 g/l, des salpetersaueren Ammoniums (0,3%), schwefelsauren Magniums (0,5 %) und saueren phosphorsauren Kaliums (0,2 %) bei Temperatur 26° C, Umrührung (300 ppm), Aeration (2 I Luft/min) innerhalb von 54 Stunden gezüchtet.

In verwandelten Milchrohstoff (Kulturflüssigkeit) gibt es sauere und neutrale Proteasen und Kollogenasen nach der Züchtung des Pilzgeflechts und nach der Abtrennung der Pilzbiomasse Die meisten werden am Ende der Inkubation, d.h. nach 36 - 54 Stunden, maximal.

Das fertige biologisch aktive Nahrungsmittel stellt eine von der Biomasse abgetrennte Kulturflüssigkeit dar. Sie besteht aus Enniatin (Antiviotikum aus der Gruppe der Membranionophoren, welche zusammen mit der Pilzentwicklung in den Wachstumsboden übergeht) 600 mg/l und Fermente:
Kollagenasen 500 U/ml, Proteasen -1,5 U/ml, Pektinasen - 0,6 U/ml. In der Molke, die während der Pilzzüchtung verwandelt wurde, hat man einen Stoff entdeckt, welcher die Cholesterinbildung hemmt.

So ermöglicht die angemeldete Erfindung es, die Eiweiß-Kohlenhydratzusammensetzung der Milch zu verändern, neue biologisch aktive Nahrungsmittel mit einem erhöhten Gehalt an wertvollen biologisch aktiven Verbindungen (Fermente, mehrfach ungesättigte Säuren, Enniatin) zu erzeugen. Diese Erfindung kann für die Kräftigung des Immunsystems des Menschen, für die Prophylaxe des methabolischen Syndroms, bei den Störungen der Funktion des Magen-Darm-Kanals und der Leber verwendet werden.

### Verwendete Informationsquellen

1. Allgemeine Arztpraxis. Herausgegeben von Prof. Denisov E.N., Prof. Movshovich B.L., Moskau, 2001
2. Heilungsnahrung. Hrgs. Prof. Menshikov F.K. und Prof. Marshak M.S., Medgiz - 1958, Moskau
3. Food product and method for producing thereof. // WO 02065844, 29.08.2002.
4. Lactic acid drink and process for the production of the same. // EP 0469200, 05.02.1992.
5. Biologisch aktiver Zusatz "Floravit E" in Gastroenterologie. Methodische Empfehlungen für Ärzte. Ausgabe der Russischen Medizinakademie für postgraduale Ausbildung, Moskau, 2002
6. Verfahren der Erzeugung des Eiweiß-Futterzusatzes. // SU 1833423 A3, 07.08.1993.
7. Präparat, welches den Gewebemetabolismus beeinflusst und Verwendung des Stammes des Pilzes Fusarium sambucinum Fuckel var ossicolum (Berk et. Curt) Bilai für seine Erzeugung. // RU 2040932, 21.12.1993.
8. Biologisch aktiver Nahrungszusatz (Ausführungsformen) und Verfahren für seine Erzeugung (Ausführungsformen) RU 2177699 C1, 27.06.2000.
9. Verfahren zur Heilung und Prophylaxe der allergischen Erkrankungen. // RU 2000808 C1, 15.10.1993 (Prototyp).

## Patentansprüche

1. Biologisch aktives Produkt auf Milchbasis, welches bei der Züchtung des Pilzes der Art Fusarium erzeugt wird,
**dadurch gekennzeichnet,**
**dass** es Kulturflüssigkeit und Biomasse enthält, die bei der Züchtung der Pilze der Art Fusarium sambucinum auf Sauer- oder Labmolke oder fettarmer Milch erzeugt wurden und bei der Feuchtigkeit von 6,5 % einen Fettgehalt von 10 -
15 % aufweist, wobei die Fett- und Säurezusammensetzung in % von der Summe der Fette beträgt:
α-Linolensäure 0,2 - 0,4 %, γ-Linolensäure 0,4 - 0,7 %, Eikosantriensäure 0,2 - 0,35 %, Arachidonsäure 0,4 - 0,8 %;
Proteine 24 - 35 %, darunter in % der Summe der Proteine Aminosäure 16 - 24%, Laktose 1 - 3 % und Asche 6 - 8 % sowie Vitamine 300 - 500 mg/100 g, darunter in mg/100 g Vitamin E 6 - 8 mg/100 g, Vitamin B1 0,6 - 0,9 mg/100 g, Vitamin B2 4 - 6 mg/100 g, Vitamin B3 3,5 - 6,5 mg/100 g und Vitamin B4 250 - 400 mg/100 g.

2. Biologisch aktives Produkt auf Milchbasis nach Anspruch 1,
dass es als Nahrungsmittel Kulturflüssigkeit und Biomasse enthält, das bei der Züchtung der Stämme des Pilzes der Art Fusarium sambucinum aus einem der Reihe Fusarium sambucinum ASM F-26109D, F-165D-WSB-916(PS-64), F-169D-WSB- 917, F-3051 D, F-3052D, F-8427D, F- 199D-52587 oder F-676D-139S genommen wird.

3. Biologisch aktives Produkt auf Milchbasis, welches bei der Züchtung des Pilzes der Art Fusarium erzeugt wird,
**dadurch gekennzeichnet,**
**dass** es von der Biomasse abgetrennte Kulturflüssigkeit enthält, die bei der Züchtung der Pilze der Art Fusarium sambucinum auf Sauer- oder Labmolke oder auf fettarmer Milch erzeugt wurde und dabei folgende Stoffe aufweist: Enniatin 100 - 800 mg/l und Fermente: Kollagenasen 100 - 800 U/ml, Proteasen 1 - 2 U/I sowie Pektinasen 0,4 - 0,8 U/ml.

4. Biologisch aktives Produkt auf Milchbasis nach Anspruch 3,
**dadurch gekennzeichnet,**
**dass** es Kulturflüssigkeit enthält, die bei der Züchtung der Stämme des Pilzes der Art Fusarium sambucinum aus einem der Reihe Fusarium sambucinum ASM F-26109D, F-165D-WSB-916(PS-64), F 169D-WSB- 917, F-3051D, F-3052D, F-8427D, F- 199D-52587 oder F-676D-139S genommen wird.
